# EUROPEAN PATENT APPLICATION

(11) **EP 1 604 702 A1**
(43) Date of publication of application: **14.12.2005**
(21) Application number: 04425417.5
(22) Date of filing: 08.06.2004
(51) Int. Cl.: A61M 25/01

(54) **A vascular guidewire for venous valvular angioplasty**

(71) Applicant: Sango S.A.S, 00139 Roma (IT)
(72) Inventor: Camilli, Sante, 00139 Roma (IT); Camilli, Daniele, 00139 Roma (IT)

(57) **Abstract**

Vascular guidewire for intravascular use, including a tip segment (1), an intermediate segment (2) and a caudal segment (3), in succession to each other. The intermediate segment includes two curvilinear flexible wires (4, 5) joined to each other by their extremities in extremal commissures (6, 7), and eventually one or more intermediate commissures (8₁), and which in their rest condition are coplanar and bent into one or more consecutive bell ansae (4₁; 4₂; 5₁, 5₂) - each one between consecutive commissures - that turn their convexity in each wire towards the same side, whilst they turn their convexity towards the opposite side relative to the bell ansae of the other wire. Application for vascular angioplasty for restoring competence to venous valves.

## Description

This invention relates to the field of instruments for vascular surgery.

More particularly, this invention relates to vascular guides or guidewires for intravascular use.

Instruments are presently available for intravascular use including an outer jacket or catheter and a moving core or guidewire, gliding in the interior thereof, such as set forth for instance in *COOK - Prodotti per Radiologia Diagnostica ed Interventistica,* William Cook Europe A/S. Such guidewires, provided in disposable sterile packages, serve in surgery and radiology, and particularly in angiography (i.e. the X-ray observation of blood-vessels), as a guide for another subsequent instrument, such as for instance a catheter, a stent, a filter, an inflateable balloon.

Such guidewires, on the other side, also find application in urological surgery, in biliary surgery for percutaneous biliary drainage, in general surgery for abscess drainage and first of all in cardiovascular surgery for percutaneous interventional treatment into cardiovascular system.

Such guidewires are made up of a flexible corewire or internal wire, gradually tapering up to a very flexible distal tip, longitudinally wound by an easy-gliding external spiral. The gap between the corewire and the outer spiral is filled with a bonding material, whilst the outer surface of the spiral can be coated through various polymeric materials. In particular, guidewires are available which are hydrophobic, or on the contrary hydrophilic, i.e. wherein the outer spiral thereof is wettable, i.e. such that water adheres thereto, favouring the gliding of guidewires themselves. Typical indicative measures for such guidewires are a length of 100 to 260 cm (39,37-102,36 inches), and a diameter of 0,36 to 0,97 mm (0,014 to 0,038 inches).

The need is felt in vascular surgery for an intravascular means useful for distending, observing and measuring a venous valvular site, fit for use in combination with angioscopy, fluoroscopy or echography, to the aim at monitoring the execution of a venous valvuloplasty. The monitoring of such valvuloplasty has not been easy heretofore, as a means for distending the vein is still not available at the present day.

The exigency of being able to dilate a vein in latero-lateral direction is particularly felt in the use of the external support to restore the competence of venous valves taught in copending European Patent Application No. 04425074.4, inventor SANTE CAMILLI, one of the Inventors hereof, having the title *External Support for Restoring Competence* to *Venous Valves by Traction of Their Intercommissural Walls,* filed on 6th February 2004. In fact, it is critical to establish which is the optimum size for the external support itself to achieve the best desired result.

Therefore, it is the object of this invention to provide an instrument for distending a vein from inside, allowing the vein to be viewed and to be analyzed by angioscopy, fluoroscopy or echography.

Such an object is reached by a guidewire for intravascular use as recited in appended Claim 1.

Preferred embodiments are set forth in dependent claims.

This invention will be fully understood based on the following detailed disclosure of its preferred embodiments, only given as a matter of example, absolutely not of restriction, referring to the annexed drawings, wherein:
*Figure 1* represents a front view of an inventive guidewire, wherein an intermediate, preceptive, segment is put into evidence in a two-eyelet embodiment thereof;
*Figures 2A, 2B, 2C, 2D, 2E* represent, in succession from the top to the bottom, outstanding cross-sections of the inventive guidewire from a tip extremity to a caudal extremity thereof, and
*Figures 3A, 3B, 3C, 3D, 3E* represent, in succession from the top to the bottom, outstanding cross-sections of the inventive guidewire from a tip extremity to a caudal extremity thereof, according to an alternate embodiment.

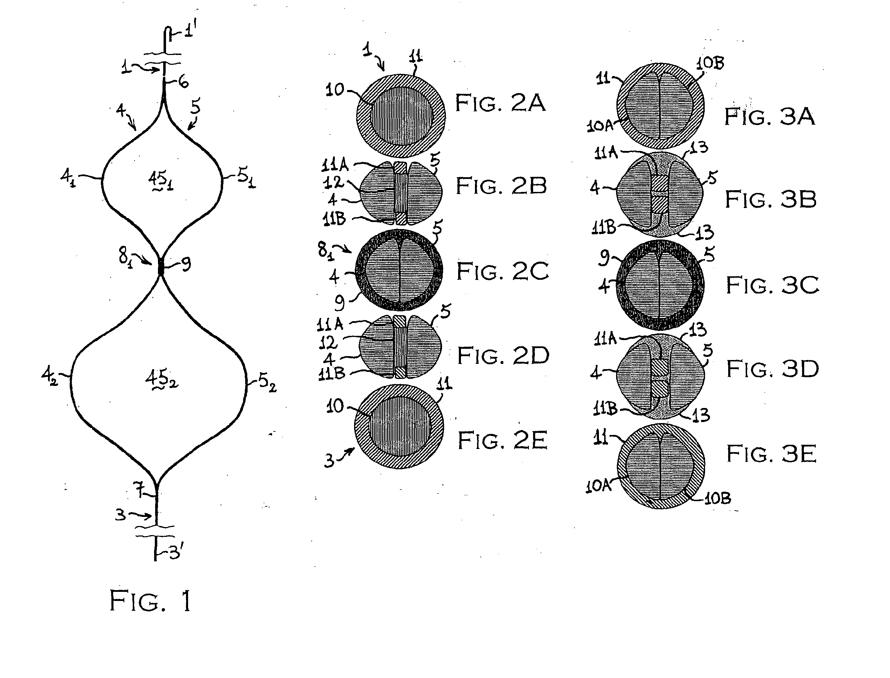

Referring to *Figure 1*, a guidewire according to this invention includes three segments, spliced in succession. A first and a third extremal segment, respectively a tip one **1** and a caudal one **3**, according to a first embodiment are built by the classical technique of angiographic guides, i.e., as mentioned above, they include a flexible corewire covered with an easy-gliding spiral, particularly in a wettable or hydrophilic material, eventually coated for instance with Teflon-like or heparin-like material; whilst a second, intermediate segment **2** is preceptive. Tip segment **1** can be terminated by a J-shaped tip **1';** caudal segment **3** can be terminated by a straight tip **3',** as known in the art of angiographic guidewires.

In intermediate segment **2** the guidewire includes two curvilinear flexible wires **4**, **5** in a high-flexibility material, such as a superelastic metal alloy, for instance Nitinol® or medical-grade steel. The two curvilinear flexible wires **4, 5,** as depicted in *Figure* *2C*, can be of a substantially semicircular cross-section, with the diameters of the semicircles faced to each other, and joined with each other by their extremities to tip and caudal segments **1, 3** respectively in a tip commissure **6** and caudal commissure **7**. The two curvilinear flexible wires **4, 5** in their rest condition are both bent, in the same plane, in one or more consecutive bell ansae that turn their convexity in each wire by the same side, whilst they turn their convexity by the opposite side relative to those of the other wire. The two flexible wires **4, 5** are solidly connected with each other at the unions between subsequent ansae by means of intermediate commissures, featuring one or more consecutive eyelets, having respective spans.

An outstanding embodiment is represented in *Figure 1* having two ansae **4**_{**1**}**, 4**_{**2**}; **5**_{**1**}; **5**_{**2**}, solidly connected through an intermediate commissure **8**_{**1**}, so two eyelets **45**_{**1**}, **45**_{**2**} remaining individuated. It is envisaged that the protrusion of the ansae, i.e. the span of the eyelets, increases in succession in caudal sense. Intermediate, preceptive segment **2**, therefore, features a protruding configuration, but by virtue of its flexibility it can be easily pushed or withdrawn and linearized inside a small-diameter linear catheter, with reciprocal collapsing of the two curvilinear flexible wires lengthwise.

Referring to *Figures 2A* to *2E*, the structure is shown of the guidewire of this invention through a succession of outstanding cross-sections thereof from tip extremity **1'** to caudal extremity **3'.** Referring to *Figures 2a* and *2E*, tip segment **1** and caudal segment **3** can be conventional, i.e. they can include an internal corewire **10** wound by a spiral **11,** for instance in the shape of a ribbon, the coils whereof are put into evidence in *Figure* *1*. Spiral **11** according to the embodiment envisaged herein is a double-wire one, i.e. it includes two spirals wound equisensely; however, the spiral is not restricted to be a two-wire one; it can also be a single-wire one.

Referring to *Figure 2C,* intermediate commissure **8**₁ is depicted between the two wires of preceptive, intermediate segment **2**. The two wires are faced to each other by their diameters and tightened by a crimped bandlet **9**, for instance in a metal, in the intermediate commissure.

The length of the guidewire is irrelevant to the ends of this invention as regards the tip and the caudal segments, whilst for the intermediate, preceptive segment, in the embodiment having two eyelets of *Figure 1* it is preferably of 30 to 40 mm (1,18 to 1,57 inches). The width of the intermediate preceptive segment between the peaks of the ansae, i.e. the span of the eyelets, in the rest condition preferably is of 8 to 10 and 12 to 15 mm respectively for the first and the second eyelet in the caudal sense **45**_{**1**}**, 45**_{**2**}**.** A third eyelet can be present in the caudal sense, having a span of 15 to 20 mm. Anyhow, for specific uses, other measures of the planar width of the intermediate segment can be used. The wires of the intermediate segment can be of a width of 1 mm and thickness of 0.5 mm. Crimped bandlet **9** can be of 1 to 3 mm in length. The maximum diameter of the guide is such that one can easily introduce and manoeuvre it inside the operative channel of an angioscope; for instance it can be of 0,97 mm (0,038 inches).

Referring to *Figures 2B* and *2D,* examples of connections are illustrated of intermediate segment **2** respectively with tip **1** and caudal **3** segments. The cylindrical corewire **10** is tapered into an attack band **12,** restricted to a width lesser than the diameters of curvilinear flexible wires **4, 5.** The attack band is inserted between these latter and is tightened between the diameters thereof, leaving residual gaps diametrically opposite to each other between the faced wires themselves. The thickness of the attack band can be of 0,2 mm, and the insertion deepness can be of up to 5 to 10 mm. Heads **11A, 11B** of double-wire spiral **11** are inserted into these residual gaps and solidly connected, for instance welded or bonded with bonding material poured into the residual interspaces between the two wires **4, 5**; otherwise, or additionally, the extremal segments of the guide wire can be solidly connected to the intermediate segment through conventional mechanical attachment means. It will be obvious to derive the modification of the constitution of the connections disclosed in the case of a single-wire winding spiral, in this case a single spiral head will be inserted in the connections.

Alternately, the connection means include a tubular mechanical engagement which bites said tip segment or said caudal segment at their heads on a side, and said tip segment on the other. The tubular mechanical engagement and the heads of said tip, caudal and intermediate segments are endowed with complementary attachment means. The heads of the wires making up the segments can be knurled or threaded.

Referring to *Figures 3A* and *3E,* in an alternate embodiment, the constitution of the double-wire intermediate, preceptive segment can also be the prosecution of the tip and caudal segments, i.e., also the internal corewire of these ones can be made up with two wires **10A**, **10B** having a susbtantially semicircular section, mated by their diameters. Double-wire intermediate segment **4, 5,** visible in *Figure 3C* - the cross-section of intermediate commissure or commissures of the intermediate segment - is connected, as depicted in *Figures 3B* and *3D*, by bringing extremities **11A, 11B** of the two windings of double-wire spiral **11** around corewire **10A, 10B,** and inserting them between extremities of wires **4, 5** of the intermediate segment. The so created commissure is solidly connected by burying it in a bonding material **13**, as can be seen in *Figures 3B* and *3D*. Alternately, or additionally, it can be solidly connected with mechanical engagement means.

Advantageously, the intermediate, preceptive segment can be equipped with markers useful for monitoring.

Having disclosed the constitution of the inventive guidewire, the way of use thereof turns out to be apparent for dilating a blood-vessel to choose the right size of the above-mentioned external support.

The inventive guide is introduced into the operative channel of an angioscope up to introduce intermediate, preceptive segment **2** thereinto. The assembly of the angioscope with the inventive guidewire loaded thereinto is pushed in bloodstream inside a vein up to a venous valve, the competence whereof is to be restored. Once a test of the incompetence of the valve accomplished, the guide is pushed, so that intermediate segment **2** goes out of the channel of the angioscope and can be dilated towards the rest condition thereof, deploying itself onto a plane inside the valvular bulb. Under angioscopic or fluoroscopic or echographic viewing, one orientates the plane of the intermediate segment parallelly to the intercommissural diameter of the valve. The intercommissural diameter of the latter is thereafter dilated by the pressure exerted by the bell ansae of the intermediate, preceptive segment, up to the restoration of the competence of the valve. This can be checked at once through the slight variation of hydrostatic gradient, such as for instance VALSALVA's manoeuvre: till blood flows back, the valve is incompetent; when blood backflow stops, the valve is restored to competence. If the first eyelet does not dilate enough, the subsequent eyelets, having increasing spans, are pushed.

Once dilated enough, the optimum intercommissural diameter can be gauged from outside, by transcutaneous echography or fluoroscopy. In this way the optimum diameter of the external support can be chosen, mentioned above, to be applied for restoring valvular competence.

At this point a surgical exposure is performed of the valvular site to be cured and said external support is applied by anchoring it to the venous wall by two pairs of opposite sutures stitches, a pair thereof being at the level of the intercommissural diameter.

This invention has been disclosed and illustrated referring to specific embodiments thereof, but it is to be expressly understood that modifications, additions and/or variations can be made, without so departing from the relevant protection scope, which is only restricted by the appended claims.

**References**

*COOK* - *Prodotti per Radiologia Diagnostica ed Interventistica,* William Cook Europe A/S.

## Claims

1. A vascular guidewire for intravascular use, including in combination: a tip segment (1), an intermediate segment (2) and a caudal segment (3), in succession to each other; said tip segment (1) and said caudal segment (3) including rectilinear internal flexible corewire means (10; 10A; 10B) wound by spiral winding means (11); and said intermediate segment (2) including two curvilinear flexible wires (4, 5; 10A; 10B), joined to each other by their extremities in a tip commissure (6) and a caudal commissure (7), and eventually in one or more intermediate commissures (8₁) between the latter, and which in their rest condition are both coplanarly bent into one or more consecutive bell ansae (4₁; 4₂; 5₁, 5₂), each one between consecutive commissures (6, 8₁, 7), that turn their convexity in each wire towards the same side, whilst they turn their convexity towards the opposite side relative to the bell ansae of the other wire; so featuring one or more consecutive eyelets (45₁, 45₂).

2. The vascular guidewire according to Claim 1, wherein said eyelets have a span that increases in caudal sense.

3. The vascular guidewire according to Claim 1 or 2, wherein said tip (1), intermediate (2) and caudal (3) segments are separate; the tip and the caudal segments respectively include an internal corewire (10); and wherein means are included for splicing the tip and caudal segments with the intermediate segment.

4. The vascular guidewire according to anyone of Claims 1 to 3, wherein said splicing means include: a tapered attack band extremity of said corewire, inserted between said two curvilinear flexible wires; the heads of said spiral winding means, inserted into residual gaps between the heads of the flexible wires and said corewire attack band extremity; and means for solidly connecting the so created splicing.

5. The vascular guidewire according to Claim 4, wherein said solidly connecting means is a welding, for instance microwelding, or a bonding material and/or an indentation provided on said corewire attack band extremity.

6. The vascular guidewire according to Claim 3, wherein said splicing means include a tubular mechanical engagement which bites said tip segment or said caudal segment at their heads on a side, and said intermediate segment on the other side, the tubular mechanical engagement and the heads of said tip, caudal and intermediate segments being endowed with complementary engagement means, such as knurling, threading, indenting.

7. The vascular guidewire according to Claim 1 or 2, wherein said tip (1) and caudal (3) segment include a double-wire corewire (10A, 10B), the two wires whereof are in continuity with said curvilinear flexible wires (10A, 10B) of said intermediate segment.

8. The vascular guidewire according to Claim 7, wherein the heads (11A, 11B) of said spiral winding means of the tip and caudal segments are fixed within the bifurcation between the two wires, in correspondence with said tip and caudal extremities of said intermediate segment, buried in pourable solidly connecting means.

9. The vascular guidewire according to anyone of the preceding claims, wherein said intermediate segment is endowed with marker means for monitoring.
